# EUROPÄISCHE PATENTSCHRIFT

(11) **EP 0 940 402 B1**
(45) Veröffentlichungstag und Bekanntmachung des Hinweises auf die Patenterteilung: **10.01.2007**
(21) Anmeldenummer: 99102584.2
(22) Anmeldetag: 11.02.1999
(51) Int. Cl.: C07D 487/22

(54) **Quarternäre polycyclische Polyammoniumsalze und Verfahren zu deren Herstellung**
Quaternary polycyclic polyammonium salts and process for their preparation
Sels de poly-ammonium quaternaires polycycliques et procédé pour leur préparation

(30) Priorität: 05.03.1998 DE 19809542
(43) Veröffentlichungstag der Anmeldung: 08.09.1999
(73) Patentinhaber: Clariant Produkte (Deutschland) GmbH, 65929 Frankfurt am Main (DE)
(72) Erfinder: Nestler, Bernd Dr., 65929 Frankfurt (DE); Seebach, Michael Dr., 65795 Hattersheim (DE)

(56) Entgegenhaltungen:
- WO-A-97/01360
- WO-A-97/49691
- WO-A-98/39335
- WEISMAN G R ET AL: "CROSS-BRIDGED CYCLAM. PROTONATION AND LI+ COMPLEXATION IN A DIAMOND-LATTICE CLEFT" JOURNAL OF THE AMERICAN CHEMICAL SOCIETY, AMERICAN CHEMICAL SOCIETY, WASHINGTON, DC, US, Bd. 112, Nr. 23, 1990, Seiten 8604-8605, XP002066456 ISSN: 0002-7863
- WEISMAN G R ET AL: "SYNTHESIS AND TRANSITION-METAL COMPLEXES OF NEW CROSS-BRIDGET TETRAAMINE LIGANDS" CHEMICAL COMMUNICATIONS, ROYAL SOCIETY OF CHEMISTRY, GB, Nr. 8, 1996, Seiten 947-948, XP001031187 ISSN: 1359-7345

## Beschreibung

Diese Erfindung betrifft spezielle quaternäre polycyclische Polyammoniumsalze mit einem Grundgerüst der allgemeinen Formel (1) 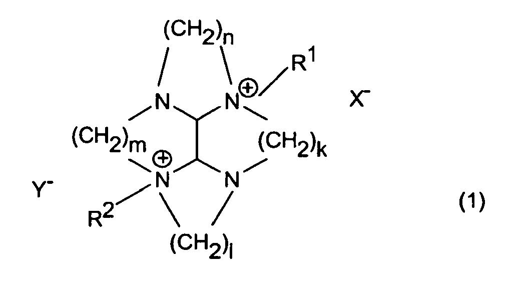 worin k, l, m und n unabhängig voneinander Zahlen von 2 bis 4 darstellen und R¹ und R² unabhängig voneinander einen substituierten oder unsubstituierten Alkyl-, Cycloalkyl- oder Arylrest und X und Y ein Anion bedeuten. Diese Verbindungen dienen als Ausgangsmaterialien für die Herstellung von cyclischen oder überbrückten Polyaminverbindungen; z.B. für Synthesen von pharmakologisch wirksamen Substanzen, von Liganden für katalytisch wirksame Metallkomplexe, von Host-Verbindungen für supra-molekulare Strukturen, von Protonenschwämmen, zudem weisen diese oftmals außergewöhnliche Redoxeigenschaften auf.

So beschreiben G. Weisman, M. Rogers, E. Wong, J. Jasinski und E. Paight in J. Am. Chem. Soc. 112 (1990), 8604-8605 die Herstellung des überbrückten Liganden II durch Reduktion von cis-4,11-Dimethyl-4,7,11,14-tetraazaperhydropyreniumdijodid (I) 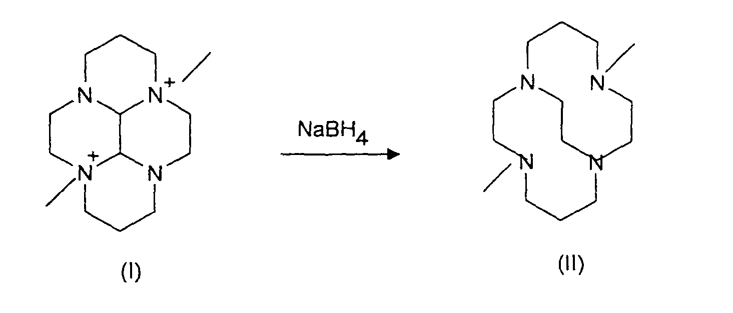 mittels Natriumborhydrid. II komplexiert mit ungewöhnlich hoher Selektivität Li⁺-Ionen und erweist sich als ein Protonenschwamm mit außergewöhnlichen Eigenschaften.

In der Literatur sind nur wenige Beispiele von quaternären polycyclischen Polyammoniumsalzen mit einem Grundgerüst der allgemeinen Formel (1) bekannt, wobei ausschließlich Diiodide und Dibromide beschrieben sind.

Diese bekannten Diiodide und Dibromide sind aber für eine Handhabung im technischen Maßstab in aller Regel ungeeignet. Neben ökologischen Aspekten ist vor allem von Nachteil, daß sich bei vielen Reaktionen (z.B. Oxidationen und Reduktionen) von Ammoniumsalzen die Anwesenheit von Halogenidionen wie Jodid- oder Bromid als störend erweist; die Folge sind oftmals unbefriedigende Ausbeuten sowie die Bildung von unerwünschten Nebenprodukten.

Dieser Erfindung liegt somit die Aufgabe zugrunde, im technischen Maßstab handhabbare quaternäre polycyclische Polyammoniumsalze mit einem Grundgerüst der allgemeinen Formel (1) zu finden.

Überraschenderweise wurde gefunden, daß quaternäre polycyclische Polyammoniumsalze mit einem Grundgerüst der allgemeinen Formel (1) als Methylsulfat- oder Sulfat-Salz nicht mit diesen Nachteilen behaftet sind, und daß diese Verbindungen zudem mit exzellenter Ausbeute in Folgereaktionen, vor allem Reduktionen mit Natriumborhydrid einsetzbar sind.

In der Literatur sind nur wenige Methoden für die Herstellung von quaternären polycyclischen Polyammoniumsalzen mit einem Grundgerüst der allgemeinen Formel (1) beschrieben, auch wurden diese lediglich zur Synthese kleiner Mengen für wissenschaftliche Zwecke erarbeitet.

In der nicht vorveröffentlichten Schrift WO 98/39335 ist erwähnt, dass man cis-Perhydro-3a,5a,8a,10a-tetraazapyren mit Alkylhalogeniden, mit Dimethylsulfat oder Methyltosylat quaternieren kann.

Die Umsetzung von cis-Perhydro-3a,5a,8a,10a-tetraazapyren mit 15 Äquivalenten Methyliodid in Acetonitril wurde von G. Weisman, M. Rogers, E. Wong, J. Jasinski und E. Paight in J. Am. Chem. Soc. 112 (1990), 1023-1033 beschrieben; nach 72 Stunden Reaktionszeit wird hierbei cis-3a,8a-Dimethyl-5a,10a-diaza-3a,8a-diazoniaperhydropyreniumdijodid in 80 % Ausbeute erhalten. Problematisch ist neben der Verwendung des giftigen Acetonitril als Solvens vor allem die lange Reaktionsdauer von 72 Stunden trotz des Einsatzes eines vielfachen Überschusses des Alkylierungsmittels Methyljodid.

Über die entsprechende Diquaternisierung des trans-Isomeren wurde von T. Okawara, H. Takaishi, Y. Okamoto, T. Yamasaki und M. Furukawa in Heterocycles 41 (1995), 1023 berichtet. Nach einer 12stündigen Behandlung von trans-Perhydro-3a,5a,8a,10a-tetraazapyren mit 10 Äquivalenten Methyliodid in Chloroform als Solvens bei 60 °C im Bombenrohr wird trans-3a,8a-Dimethyl-5a,10a-diaza-3a,8a-diazoniaperhydropyreniumdijodid in einer Ausbeute von 67 % erhalten. Auch hier ist die für die Umsetzung notwendige Dauer von 12 Stunden bei Verwendung eines fünffachen Überschusses an Alkylierungsmittel und einer Reaktionstemperatur von 60 °C nicht befriedigend.

Eine Reihe von Diquaternisierungen mit Benzylbromid als Alkylierungsmittel wurde von G. Weisman, E. Wong, D. Hill, M. Rogers, D. Reed und J. Calabrese in Chem. Commun. 1996, 947-948 untersucht. Demnach ergibt die Umsetzung von cis-Perhydro-3a,5a,8a,10a-tetraazapyren mit überschüssigem Benzylbromid in Acetonitril bei Raumtemperatur nach einer Reaktionszeit von 3 bis 21 Tagen cis-3a,8a-Dibenzyl-5a,10a-diaza-3a,8a-diazoniaperhydropyreniumdibromid in 78 - 99 %-iger Ausbeute.

Diese bekannten Verfahren zur Herstellung der gewünschten quaternären polycyclischen Polyammoniumsalze mit einem Grundgerüst der allgemeinen Formel (1) haben mehrere Nachteile: Die erzielbaren Ausbeuten liegen z.T. bei nur 67 %, als Lösungsmittel wird das giftige Acetonitril bzw. Chloroform eingesetzt, als Alkylierungsmittel werden im Hinblick auf den Umweltschutz bedenkliche Brom- und Iod-organische Verbindungen eingesetzt. Verwendet wird zudem ein großer Überschuß dieser Reagentien, was die Isolierung der gewünschten Produkte erschwert. Zudem sind durchweg lange Reaktionszeiten von 12 Stunden bei 60 °C bis zu 21 Tagen bei Raumtemperatur notwendig, so daß diese Methoden zur Herstellung technischer Mengen dieser quaternären polycyclischen Polyammoniumsalze ungeeignet sind.

Dieser Erfindung liegt deshalb außerdem die Aufgabe zugrunde, ein einfaches Verfahren zur Synthese von quaternären polycyclischen Polyammoniumsalzen mit einem Grundgerüst der allgemeinen Formel (1) zu finden. Desweiteren soll das erfindungsgemäße Verfahren zur Herstellung technischer Mengen geeignet sein, aus toxikologischer Sicht bedenkliche Lösungsmittel vermeiden, zudem sollen die entstehenden Produkte in hoher Ausbeute mit minimalen Abfallmengen anfallen.

Überraschenderweise wurde gefunden, daß, wenn nicht Brom- oder Iod-organische Verbindungen, sondern andere zur Übertragung von Alkylgruppen geeignete Substanzen, insbesondere Dialkylsulfate und Ester von organischen Sulfonsäuren als Alkylierungsmittel verwendet werden, außerordentlich kurze Reaktionszeiten für die Synthese von quaternären polycyclischen Polyammoniumsalzen mit einem Grundgerüst der allgemeinen Formel (1) ausreichen.

Gegenstand der Erfindung sind quaternäre polycyclische Polyammoniumsalze der allgemeinen Formel (2) 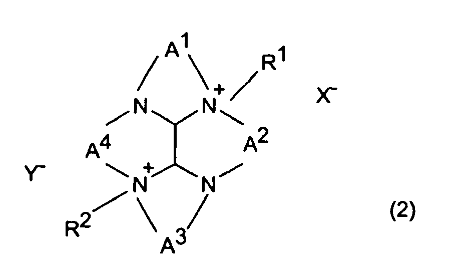 worin
- A¹, A², A³, und A⁴: unabhängig voneinander entweder einen C₂- bis C₄-Alkylenrest, der durch eine oder mehrere Gruppen P und/oder Q substituiert sein kann, wobei
- P: eine C₁- bis C₃₀-Alkylgruppe, die mit einer oder mehreren Gruppen Q substituiert sein kann, und
- Q: -eine Gruppe COR, worin R eine Hydroxy-, eine C₁- bis C₅-Alkoxy-oder C₆- bis C₁₄-Aryloxy- oder eine unsubstituierte Aminogruppe,
-eine C₆- bis C₁₄-Arylgruppe, die substituiert sein kann mit einer oder mehreren C₁- bis C₃₀-Alkyl-, C₁- bis C₅-Alkoxy- oder C₆- bis C₁₄-Aryloxy-,
unsubstituierten Aminogruppen, Halogenatomen,
Cyanogruppen, Sulfogruppen, Carboxylgruppen oder
- Gruppen der Formel -(CH₂)ᵣ-COOH, -(CH₂)ᵣ-SO₃H,
- (CH₂)ᵣ-PO₃H_{2,} -(CH₂)ᵣ OH, wobei r eine ganze Zahl von 0 bis 4 bedeutet und die genannten Säuregruppen auch in Salzform vorliegen können,
- eine substituierte oder unsubstituierte Aminogruppe,
- eine Hydroxygruppe,
- eine C₁- bis C₃₀-Alkylgruppe,
- eine C₁- bis C₃₀-Alkoxy- oder C₆- bis C₁₄-Aryloxygruppe,
- ein Halogenatom,
- eine Cyano-, Sulfo- oder Carboxylgruppe,
- eine Gruppierung der Formel -(CH₂)ᵣ-COOH, -(CH₂)ᵣ-SO₃H, -(CH₂)ᵣ-PO₃H₂, -(CH₂)ᵣ OH, wobei r eine ganze Zahl von 0 bis 4 bedeutet und die genannten Säuregruppen auch in Salzform vorliegen können;
- oder A¹, A², A³ und A⁴: eine Gruppe -(CH₂)ₛ-E-(CH₂)ₜ-, worin E für einen C₆- bis C₁₄-Arylenrest, der mit Gruppen P und/oder Q substituiert sein kann, wobei den Gruppen P und Q die oben genannte Bedeutung zukommt und s und t unabhängig voneinander die Werte 0, 1 oder 2 einnehmen können,
- R¹ und R²: unabhängig voneinander eine C₁- bis C₁₆-Alkylgruppe bedeutet, die mit einem oder mehreren C₁- bis C₂₀-Alkylresten, C₁- bis C₄-Alkoxygruppen, Halogenatomen, Sulfogruppen, Carboxylgruppen oder Gruppierungen der Formel -(CH₂)_{w}-COOH, -(CH₂)_{w}-SO₃H, -(CH₂)_{w}-PO₃H₂, -(CH₂)_{w}-OH, wobei w eine ganze Zahl von 0 bis 5 bedeutet und die genannten Säuregruppen auch in Salzform vorliegen können, substituiert sein kann und
- X⁻ und Y⁻: unabhängig voneinander R³SO₄⁻ und SO₄²⁻ bedeuten, wobei R³ jeweils für Wasserstoff, C₁- bis C₈-Alkyl oder C₆- bis C₁₈-Aryl, gegebenenfalls substituiert mit C₁- bis C₄-Alkyl steht, ausgenommen Methosulfat.

Bevorzugt sind Verbindungen der Formel (2), worin A¹, A², A³ und A⁴ unabhängig voneinander einen C₂-C₄-Alkylenrest, R¹ und R² unabhängig voneinander eine C₁-C₁₆-Alkyl-, insbesondere eine Methylgruppe, eine Cycloalkyl- oder Arylgruppe und X- und Y⁻ HSO₄⁻ bedeuten.

Gegenstand der Erfindung ist außerdem ein Verfahren zur Herstellung der Verbindungen der Formel (2), wobei A¹, A², A³, A⁴, R¹, R², X und Y die oben genannten Bedeutungen haben. Dieses Verfahren ist dadurch gekennzeichnet, daß man ein Polyamin der Formel (3) 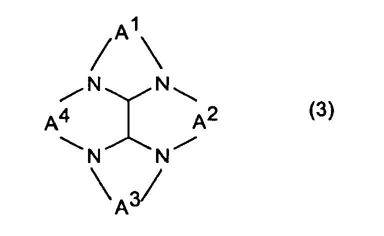 wobei A¹, A², A³ und A⁴ die oben angegebenen Bedeutungen haben, mit einer Verbindung der Formel 4a bzw. 4b

R¹ - Z (4a) bzw. R² - Z (4b)

umsetzt, wobei R¹ und R² die oben angegebenen Bedeutungen hat und Z die gleiche Bedeutung hat wie X und Y.

Beispiele für erfindungsgemäße quaternäre polycyclische Polyammoniumsalze der allgemeinen Formel 2 sind:
2a,6a-Dimethyl-decahydro-2a,4a,6a,8a-tetraaza-cyclopenta[f,g]acenaphtylen-dimethylsulfat
2a,6a-Dimethyl-decahydro-2a,4a,6a,8a-tetraaza-cyclopenta[f,g]acenaphtylen-sulfat
2a,7a-Dimethyl-decahydro-2a,4a,7a,9a-tetraaza-cyclopenta[c,d]phenalen-dimethylsulfat
2a,7a-Diethyl-decahydro-2a,4a,7a,9a-tetraaza-cyclopenta[c,d]phenalen-di-ethylsulfat
2a,7a-Dibenzyl-decahydro-2a,4a,7a,9a-tetraaza-cyclopenta[c,d]phenalen-sulfat
3a,8a-Dimethyl-decahydro-3a,5a,8a,11a-tetraaza-cyclohepta[d,e,f]phenanthren-dimethylsulfat
3a,8a-Di-cyclohexyl-decahydro-3a,5a,8a,11a-tetraaza-cyclohepta[d,e,f]phen-anthrensulfat
3a,8a-Diphenyl-decahydro-3a,5a,8a,11a-tetraaza-cyclohepta[d,e,f]phenanthrensulfat
3a,8a-Diethyl-decahydro-3a,5a,8a,10a-tetraazapyren-di-ethylsulfat
3a,8a-Diethyl-decahydro-3a,5a,8a,10a-tetraazapyren-sulfat
3a,8a-Dibenzyl-decahydro-3a,5a,8a,10a-tetraazapyren-sulfat
3a,8a-Diphenyl-decahydro-3a,5a,8a,10a-tetraazapyren-sulfat
3a,9a-Dimethyl-decahydro-3a,6a,9a,12a-tetraaza-dibenzo[ef, kl]heptalen-dimethylsulfat
3a,9a-Dibenzyl-decahydro-3a,6a,9a,12a-tetraaza-dibenzo[ef, kl]heptalen-sulfat
3a,9a-Di-(methoxymethyl)-decahydro-3a,6a,9a,12a-tetraaza-dibenzo[ef, kl]heptalensulfat,
wobei jeweils die cis- und trans-Isomere sowie Isomerengemische gemeint sind. Besonders bevorzugt sind von Decahydro-3a,5a,8a,10a-tetraazapyren abgeleitete biquaternäre polycyclische Polyammoniumsalze.

Die als Ausgangsstoffe für das erfindungsgemäße Verfahren dienenden polycyclischen Polyamine der Formel 3 können, wie z.B. von G. Weisman, E. Wong, D. Hill, M. Rogers, D. Reed und J. Calabrese in Chem. Commun. 1996, 947 - 948 beschrieben, durch Kondensation von Glyoxal mit cyclischen Tetraaminen hergestellt werden.

Beispiele für Grundgerüste derartiger polycyclischer Polyamine 3 sind
cis- und trans-Decahydro-2a,4a,6a,8a-tetraazacyclopenta[f,g]acenaphtylen,
cis- und trans-Decahydro-2a,4a,7a,9a-tetraazacyclopenta[c,d]phenalen,
cis- und trans-Decahydro-3a,5a,8a,11a-tetraazacyclohepta[def]phenanthren oder
cis- und trans-Decahydro-3a,6a,9a,12a-tetraazadibenzo[ef,kl]heptalen.

Bei den als Ausgangsstoffe für das erfindungsgemäße Verfahren dienenden Verbindungen der Formel 4a bzw. 4b werden solche Verbindungen eingesetzt, die mit tertiären Aminen zu quaternären Ammoniumverbindungen reagieren. Beispiele für derartige Reaktionen bzw. Reagentien 4a und 4b sind in J. Goerdeler AMethoden zur Herstellung und Umwandlung von quartären Ammoniumverbindungen@, Methoden der Organischen Chemie (Houben-Weyl), E. Müller (Hrsg.), Bd. XI/2 (1958), S. 587 - 640 und der dort zitierten Literatur gegeben. Bevorzugte Reagentien 4a und 4b sind hierbei insbesondere Ester der Schwefelsäure und der Sulfonsäuren wie z.B. Dialkylsulfate, Arylalkylsulfate oder Ester von organischen Sulfonsäuren wie z.B. Toluol- oder Methan-sulfonsäuremethylester.

Die Umsetzung erfolgt üblicherweise in Gegenwart eines Lösungsmittels, es ist hierbei auch möglich, das Reagenz 4a oder 4b als Lösungsmittel zu verwenden. Bevorzugt sind hierbei Lösungsmittel die unter den Bedingungen der Umsetzung nicht mit den Verbindungen 3 oder 4a und 4b reagieren, weniger geeignet sind daher z.B. Carbonsäuren oder Amine.

Üblicherweise wird für die Reaktion soviel Lösungsmittel eingesetzt, daß sich die Verbindungen 3 und 4a bzw. 4b darin lösen. Sollte keine vollständige Lösung erfolgen, kann auch in Dispersion (Suspension bzw. Emulsion) gearbeitet werden. Die Konzentration an Verbindung der Formel 3 liegt üblicherweise im Bereich von 0,01 bis 5,0 mol pro Liter Lösungsmittel, bevorzugt 0,05 bis 3,5 mol/l, besonders bevorzugt 0,1 bis 2,0 mol/l.

Die Reaktion erfolgt bei Temperaturen von 0 bis 200°C, bevorzugt 10 bis 150°C. Der hierfür benötigte Zeitraum beträgt, in Abhängigkeit von der gewählten Temperatur etwa ein bis 12 Stunden.

Zur Durchführung des erfindungsgemäßen Verfahrens wird im allgemeinen die Verbindung 3, gegebenenfalls mit dem Lösungsmittel vorgelegt und anschließend das Reagenz 4a oder 4b zugesetzt, es kann sich jedoch auch als vorteilhaft erweisen, die Verbindung 4a oder 4b und gegebenenfalls ein Lösungsmittel vorzulegen und die Verbindung 3, ggf. in einem Lösungsmittel gelöst zuzusetzen; dies ist vor allem angebracht, wenn das intermediär gebildete monoquaternäre polycyclische Polyammoniumsalz, d.h. die Vorstufe der diquaternären polycyclischen Polyammoniumverbindung der allgemeinen Formel (2), im verwendeten Reaktionsmedium nur schlecht löslich ist.

Da die Umsetzung in aller Regel exothermen Charakter hat, kann es sich als notwendig erweisen, die Reaktionslösung vor, während und/oder nach dem Zusammengeben von 3 und 4a bzw. 4b zu kühlen. Unabhängig hiervon kann es jedoch notwendig sein, nach dem Zusammengeben von 3 und 4a bzw. 4b die Reaktion durch Erwärmen zu vervollständigen.

Nach Beendigung der Reaktion wird das gebildete quaternäre polycyclische Polyammoniumsalz der allgemeinen Formel (2) aus dem Reaktionsgemisch mit den üblichen Verfahren isoliert. Geeignete Verfahren sind z.B. Filtration, Extraktion, destillative, chromatographische und osmotische Verfahren, sowie Kombinationen dieser Verfahren.

Zur Vermeidung von Nebenreaktionen kann die Umsetzung und ggf. die Aufarbeitung unter Schutzgas erfolgen, üblicherweise Stickstoff.

Bei den Verbindungen der Formel 2 sind solche bevorzugt, wo das Gegenion ein Hydrogensulfat-, Alkyl- und Arylsulfat- oder -sulfonat-Anion ist, ausgenommen Methosulfat.

Diese Verbindungen der Formel 2 ergeben überraschenderweise bei der Umsetzung mit Natriumborhydrid höhere Ausbeuten als die analogen Halogenid-Salze.

Das erfindungsgemäße Verfahren, das auch die Herstellung dieser Halogenid-Salze mit umfaßt, zeichnet sich aus durch den Anfall geringer Mengen an Abfall, sehr gute Ausbeuten und durch einfach zugängliche, zumTeil auch kommerziell in großer Menge zugänglicher Ausgangsstoffe.

### Beispiel 1 Synthese von trans-3a,8a-Dimethyl-5a,10a-diaza-3a,8a-diazoniaperhydro-pyrenium-di-methylsulfat

Zu einer Lösung von 11,1 g trans-Perhydro-3a,5a,8a,10a-tetraazapyren in 160 ml Ethanol wurden unter Kühlung 18,9 g Dimethylsulfat zugetropft, anschließend wurde zwei Stunden unter Rückfluß zum Sieden erhitzt. Die beim Abkühlen auf Raumtemperatur ausgefallenen Kristalle wurden abgesaugt und mit 50 ml Ethanol gewaschen. Nach Trocknen im Vakuum wurden 9,61 g farblose Kristalle erhalten.

### Beispiel 2 Synthese von trans-3a,8a-Dimethyl-5a,10a-diaza-3a,8a-diazoniaperhydro-pyrenium-di-methylsulfat

200 ml Dimethylsulfat wurden innerhalb von 30 Minuten portionsweise mit 44,5 g trans-Perhydro-3a,5a,8a,10a-tetraazapyren versetzt und das Gemisch 8 Stunden bei Raumtemperatur gerührt. Der ausgefallene Niederschlag wurde abgesaugt und nach Waschen mit 100 ml Aceton im Vakuum getrocknet. Man erhielt 43,5 g eines farblosen, kristallinen Feststoffs.

### Beispiel 3 Umsetzung von cis-3a,8a-Dimethyl-5a,10a-diaza-3a,8a-diazoniaperhydropyrenium-di-methylsulfat mit Natriumborhydrid

4,75 g cis-3a,8a-Dimethyl-5a,10a-diaza-3a,8a-diazoniaperhydro-pyrenium-dimethylsulfat wurden in 30 ml eines Gemisches aus Ethanol/Wasser (80:20) vorgelegt. Zu der Lösung wurden portionsweise 2,25 g Natriumborhydrid zugegeben, und anschließend 48 Stunden bei Raumtemperatur nachgerührt. Unter Eiskühlung wurden zu der Lösung 2,85 g Salzsäure (37 %) zugetropft, wobei eine weiße Suspension entstand, die am Rotationsverdampfer auf ein Volumen von 10 ml eingeengt wurde. Nach Abkühlen des Rückstandes auf 5°C wurde mit 8 ml einer 10 M Kaliumhydroxid-Lösung versetzt, viermal mit Toluol extrahiert und die vereinigten Toluolphasen bis zur Trockene eingeengt. Hierbei wurden 2,1 g (82 %) 4,11-Dimethyl-1,4,8,11-tetraaza-bicyclo[6.6.2]hexadecan in Form eines gelblichen Öles erhalten.

### Beispiel 4 Umsetzung von cis-3a,8a-Dimethyl-5a,10a-diaza-3a,8a-diazonia-perhydro-pyrenium-diiodid mit Natriumborhydrid (Vergleichsversuch zu Beispiel 3)

Zu einer heterogenen Mischung von 3,2 g cis-3a,8a-Dimethyl-5a,10a-diaza-3a,8a-diazonia-perhydro-pyrenium-diiodid in 250 ml Ethanol wurden unter Rühren langsam 7,2 g Natriumborhydrid zugegeben. Nach 72 Stunden Rühren wurde unter vorsichtiger Zugabe von 10 %iger Salzsäure leicht sauer gestellt und mit 200 ml Ethanol verdünnt. Das Lösungsmittel wurde am Rotationsverdampfer entfernt, der verbleibende Rückstand in 400 ml Wasser aufgelöst und durch Zugabe von 40 %iger Kalilauge ein pH-Wert von 14 eingestellt. Die alkalische Lösung wurde viermal mit jeweils 300 ml Toluol extrahiert, die organischen Phasen über wasserfreiem Natriumsulfat getrocknet und das Lösemittel im Vakuum entfernt. Die Destillation des Rückstandes ergab 1,0 g (62 %) 4,11-Dimethyl-1,4,8,11-tetraazabicyclo[6.6.2]hexa-decan als hellgelbes Öl. weiße Suspension entstand, die am Rotationsverdampfer auf ein Volumen von 10 ml eingeengt wurde. Nach Abkühlen des Rückstandes auf 5 °C wurde mit 8 ml einer 10 M Kaliumhydroxid-Lösung versetzt, viermal mit Toluol extrahiert und die vereinigten Toluolphasen bis zur Trockene eingeengt. Hierbei wurden 2,1 g (82 %) 4,11-Dimethyl-1,4,8,11-tetraaza-bicyclo[6.6.2]hexadecan in Form eines gelblichen Öles erhalten.

### Beispiel 4 Umsetzung von cis-3a,8a-Dimethyl-5a,10a-diaza-3a,8a-diazonia-perhydro-pyrenium-diiodid mit Natriumborhydrid (Vergleichsversuch zu Beispiel 3)

Zu einer heterogenen Mischung von 3,2 g cis-3a,8a-Dimethyl-5a,10a-diaza-3a,8a-diazonia-perhydro-pyrenium-diiodid in 250 ml Ethanol wurden unter Rühren langsam 7,2 g Natriumborhydrid zugegeben. Nach 72 Stunden Rühren wurde unter vorsichtiger Zugabe von 10 %iger Salzsäure leicht sauer gestellt und mit 200 ml Ethanol verdünnt. Das Lösungsmittel wurde am Rotationsverdampfer entfernt, der verbleibende Rückstand in 400 ml Wasser aufgelöst und durch Zugabe von 40 %iger Kalilauge ein pH-Wert von 14 eingestellt. Die alkalische Lösung wurde viermal mit jeweils 300 ml Toluol extrahiert, die organischen Phasen über wasserfreiem Natriumsulfat getrocknet und das Lösemittel im Vakuum entfernt. Die Destillation des Rückstandes ergab 1,0 g (62 %) 4,11-Dimethyl-1,4,8,11-tetraazabicyclo[6.6.2]hexa-decan als hellgelbes Öl.

## Patentansprüche

1. Quaternäre polycyclische Polyammoniumsalze der allgemeinen Formel (2) 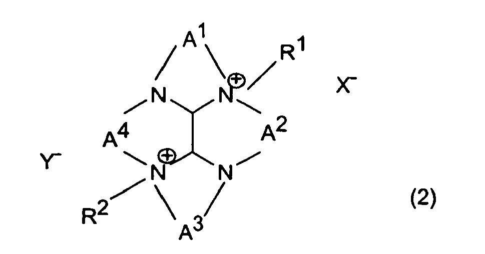 worin
A¹, A², A³, und A⁴ unabhängig voneinander entweder einen C₂- bis C₄-Alkylenrest, der durch eine oder mehrere Gruppen P und/oder Q substituiert sein kann, wobei
P eine C₁- bis C₃₀-Alkylgruppe, die mit einer oder mehreren Gruppen Q substituiert sein kann, und
Q -eine Gruppe COR, worin R eine Hydroxy-, eine C₁- bis C₅-Alkoxy- oder C₆- bis C₁₄-Aryloxy- oder eine unsubstituierte Aminogruppe, -eine C₆- bis C₁₄-Arylgruppe, die substituiert sein kann mit einer oder mehreren C₁- bis C₃₀-Alkyl-, C₁- bis C₅-Alkoxy- oder C₆- bis C₁₄-Aryloxy-, unsubstituierten Aminogruppen, Halogenatomen, Cyanogruppen, Sulfogruppen, Carboxylgruppen oder
- Gruppen der Formel -(CH₂)ᵣ-COOH, -(CH₂)ᵣ-SO₃H,
- (CH₂)ᵣ-PO₃H_{2,} -(CH₂)ᵣ OH, wobei r eine ganze Zahl von 0 bis 4 bedeutet und die genannten Säuregruppen auch in Salzform vorliegen können,
- eine unsubstituierte Aminogruppe,
- eine Hydroxygruppe,
- eine C₁-bis C₃₀-Alkylgruppe,
- eine C₁-bis C₃₀-Alkoxy- oder C₆- bis C₁₄-Aryloxygruppe,
- ein Halogenatom,
- eine Cyano-, Sulfo- oder Carboxylgruppe,
- eine Gruppierung der Formel -(CH₂)ᵣ -COOH, -(CH₂)ᵣ-SO₃H,
- (CH₂)ᵣ-PO₃H₂, -(CH₂)ᵣ OH, wobei r eine ganze Zahl von 0 bis 4 bedeutet und die genannten Säuregruppen auch in Salzform vorliegen können;
oder A¹, A², A³ und A⁴ eine Gruppe -(CH₂)ₛ-E-(CH₂)ₜ-, worin E für einen C₆- bis C₁₄-Arylenrest, der mit Gruppen P und/oder Q substituiert sein kann, wobei den Gruppen P und Q die oben genannte Bedeutung zukommt und s und t unabhängig voneinander die Werte 0, 1 oder 2 einnehmen können,
R¹ und R² unabhängig voneinander eine C₁- bis C₁₆-Alkyl-Gruppe bedeutet, die mit einem oder mehreren C₁- bis C₂₀-Alkylresten, C₁- bis C₄-Alkoxygruppen, Halogenatomen, Sulfogruppen, Carboxylgruppen oder Gruppierungen der Formel -(CH₂)_{w}-COOH, -(CH₂)_{w}-SO₃H, -(CH₂)_{w}-PO₃H₂, -(CH₂)_{w}-OH, wobei w eine ganze Zahl von 0 bis 5 bedeutet und die genannten Säuregruppen auch in Salzform vorliegen können, substituiert sein kann und
X- und Y⁻ unabhängig voneinander R³SO₄⁻ und SO₄²⁻ bedeuten, wobei R³ jeweils für Wasserstoff, C₁- bis C₈-Alkyl oder C₆- bis C₁₈-Aryl, gegebenenfalls substituiert mit C₁- bis C₄-Alkyl steht, ausgenommen Methosulfat.

2. Verbindungen nach Anspruch 1, **dadurch gekennzeichnet, dass** R¹ und R² jeweils eine C₁- bis C₁₆-Alkylgruppe bedeuten.

3. Verbindungen nach Anspruch 1, **dadurch gekennzeichnet, dass** R¹ und R² jeweils eine Methylgruppe bedeuten.

4. Verbindungen nach Anspruch 1, **dadurch gekennzeichnet, dass** A¹, A², A³ und A⁴ unabhängig voneinander jeweils einen C₂- bis C₄-Alkylenrest bedeuten.

5. Verbindungen nach Anspruch 1, **dadurch gekennzeichnet, dass** A¹ und A² einen C₃-Alkylenrest, und A³ und A⁴ einen C₂-Alkylenrest bedeuten.

6. Verbindungen nach Anspruch 1, **dadurch gekennzeichnet, dass** X- und Y⁻ ein R³SO₄⁻, HSO₄⁻ und/oder SO₄²⁻-lon bedeutet, wobei R³ Wasserstoff oder C₁- bis C₈-Alkyl bedeutet.

7. Verbindungen nach Anspruch 1, **dadurch gekennzeichnet, dass** X- und Y⁻ ein HSO₄⁻-lon bedeutet.

8. Verfahren zur Herstellung von Verbindungen nach Anspruch 1, wobei A¹, A², A³, A⁴, R¹, R², X und Y die in Anspruch 1 angegebenen Bedeutungen haben, **dadurch gekennzeichnet, dass** man ein Polyamin der Formel (3) 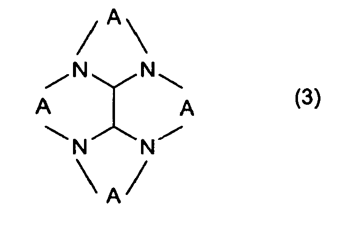 wobei A die oben für A¹, A², A³ und A⁴ angegebenen Bedeutungen hat, mit einer Verbindung der Formel 4a bzw. 4b
R¹ - Z (4a)
bzw.
R² - Z (4b)
umsetzt, wobei R¹ und R² die oben angegebenen Bedeutungen haben und Z die gleiche Bedeutung hat wie X und Y.

9. Verfahren nach Anspruch 9, **dadurch gekennzeichnet, dass** man die Umsetzung in Gegenwart der Verbindung der Formeln 4a bzw. 4b als Lösemittel durchführt.

10. Verfahren nach Anspruch 9, **dadurch gekennzeichnet, dass** pro Liter Lösemittel 0,01 bis 5,0 mol, bevorzugt 0,05 bis 3,5 mol, insbesondere bevorzugt 0,1 bis 2,0 mol der Verbindung der Formel 3 eingesetzt werden.

11. Verfahren nach Anspruch 9, **dadurch gekennzeichnet, dass** die Umsetzung bei einer Temperatur von 0 bis 200 °C, bevorzugt 10 bis 150 °C erfolgt.

## Claims

1. A quaternary polycyclic polyammonium salt of the general formula (2) 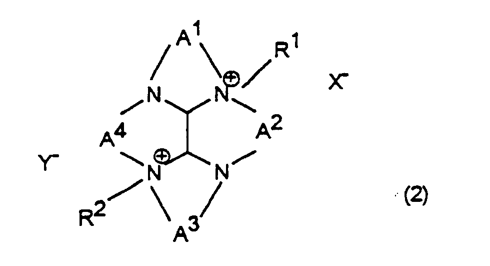 in which
A¹, A², A³ and A⁴ independently of one another are either
a C₂- to C₄-alkylene radical which can be substituted by one or more groups P and/or Q, where
P is a C₁- to a C₃₀-alkyl group which can be substituted by one or more groups Q, and
Q - is a group COR, in which R is a hydroxyl group, a C₁- to C₅-alkoxy group or C₆- to C₁₄-aryloxy group or an unsubstituted amino group,
-a C₆- to C₁₄-aryl group which can be substituted by one or more C₁- to C₃₀-alkyl, C₁- to C₅-alkoxy or C₆- to C₁₄-aryloxy groups, unsubstituted amino groups, halogen atoms, cyano groups, sulfo groups, carboxyl groups or methosulfate.

2. A compound as claimed in claim 1, wherein R¹ and R² are each a C₁- to C₁₆-alkyl group.

3. A compound as claimed in claim 1, wherein R¹ and R² are each a methyl group.

4. A compound as claimed in claim 1, wherein A¹, A², A³ and A⁴ independently of one another are each a C₂- to C₄-alkylene radical.

5. A compound as claimed in claim 1, wherein A¹ and A² are a C₃-alkylene radical and A³ and A⁴ are a C₂-alkylene radical.

6. A compound as claimed in claim 1, wherein X- and Y⁻ are an R³SO₄⁻, HSO₄⁻ and/or SO₄²⁻ ion, where R³ is hydrogen or C₁- to C₈-alkyl.

7. A compound as claimed in claim 1, wherein X⁻ and Y⁻ are an HSO₄⁻ ion.

8. A process for the preparation of compounds as claimed in claim 1, where A¹, A², A³, A⁴, R¹, R², X and Y have the meanings indicated in claim 1, which comprises reacting a polyamine of the formula (3) 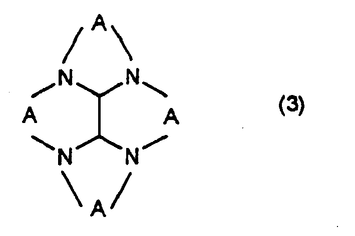 where A has the meanings indicated above for A¹, A², A³ and A⁴ , with a compound of the formula 4a or 4b
R¹-Z (4a)
or
R²-Z (4b)
where R¹ and R² have the meanings indicated above and Z has the same meaning as X and Y.
- groups of the formula -(CH₂)ᵣ-COOH, -(CH₂)ᵣ-SO₃H,
- (CH₂)ᵣ-PO₃H₂, -(CH₂)ᵣOH, where r is an integer from 0 to 4 and the acid groups mentioned can also be present in salt form,
- an unsubstituted amino group,
- a hydroxyl group,
- a C₁- to C₃₀-alkyl group,
- a C₁- to C₃₀-alkoxy or C₆- to C₁₄-aryloxy group,
- a halogen atom,
- a cyano, sulfo or carboxyl group,
- a group of the formula - -(CH₂)ᵣ-COOH, -(CH₂)ᵣ-SO₃H,
- (CH₂)ᵣ-PO₃H₂, - (CH₂)ᵣOH, where r is an integer from 0 to 4 and the acid groups mentioned can also be present in salt form;
or A¹, A², A³ and A⁴ are a group -(CH₂)ₛ-E-(CH₂)ₜ-, in which E is a C₆- to C₁₄-arylene radical which can be substituted by groups P and/or Q, where the groups P and Q have the abovementioned meaning and s and t independently of one another can assume the values 0, 1 or 2,
R¹ and R² independently of one another are a C₁- to C₁₆-alkyl group which can be substituted by one or more C₁- to C₂₀-alkyl radicals, C₁- to C₄-alkoxy groups, halogen atoms, sulfo groups, carboxyl groups or groups of the formula-(CH₂)_{w}-COOH, -(CH₂)_{w}-SO₃H, - (CH₂)_{w}-PO₃H₂, -(CH₂)_{w}-OH, where w is an integer from 0 to 5 and the acid groups mentioned can also be present in salt form, and
X⁻ and Y⁻ independently of one another are R³SO₄⁻ and SO₄²⁻, where R³ in each case is hydrogen, C₁-to C₈-alkyl or C₆- to C₁₈-aryl, optionally substituted by C₁- to C₄-alkyl, excluding

9. The process as claimed in claim 9, wherein the reaction is carried out in the presence of the compound of the formula 4a or 4b as a solvent.

10. The process as claimed in claim 9, wherein, per liter of solvent, 0.01 to 5.0 mol, preferably 0.05 to 3.5 mol, particularly preferably 0.1 to 2.0 mol, of the compound of the formula 3 are employed.

11. The process as claimed in claim 9, wherein the reaction is carried out at a temperature from 0 to 200°C, preferably 10 to 150°C.

## Revendications

1. Sels de polyammonium quaternaire polycyclique de formule générale (2) 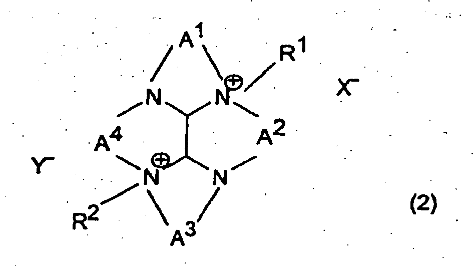 dans laquelle
A¹, A², A³ et A⁴, indépendamment l'un de l'autre, représentent
un radical alkylène en C₂ à C₄, lequel peut être substitué par un ou plusieurs groupes P et/ou Q,
P représente un groupe alkyle en C₁ à C₃₀, pouvant être substitué par un ou plusieurs groupes Q, dans lesquels
Q - représente un groupe COR, dans lequel R représente un groupe hydroxy, un groupe alcoxy en C₁ à C₅ ou un groupe aryloxy en C₆ à C₁₄ ou un groupe amino non substitué,
- un groupe aryle en C₆ à C₁₄ pouvant être substitué avec un ou plusieurs groupes alkyle en C₁ à C₃₀, alcoxy en C₁ à C₅ ou aryloxy en C₆ à C₁₄, groupes amino non substitués, atomes d'halogène, groupes cyano, groupes sulfo, groupes carboxyle ou
- des groupes de formule -(CH₂)ᵣ-COOH, -(CH₂)ᵣ-SO₃H, - (CH₂)ᵣ-PO₃H₂, -(CH₂)ᵣOH, r représentant un nombre entier de 0 à 4 et les groupes acides indiqués pouvant également se présenter sous la forme de sel,
- un groupe amino non substitué,
- un groupe hydroxy,
- un groupe alkyle en C₁ à C₃₀,
- un groupe alcoxy en C₁ à C₃₀ ou aryloxy en C₆ à C₁₄,
- un atome d'halogène,
- un groupe cyano, sulfo ou carboxyle,
- un groupement de formule -(CH₂)ᵣ-COOH, - (CH₂)ᵣ-SO₃H, -(CH₂)r-PO₃H₂, -(CH₂)ᵣ OH, r représentant un nombre entier de 0 à 4 et les groupes acides indiqués pouvant également se présenter sous la forme de sel ;
ou bien A¹, A², A³ et A⁴ représentent un groupe - (CH₂)ₛ-E-(CH₂)ₜ-, dans lequel E représente un radical arylène en C₆ à C₁₄ qui peut être substitué par des groupes P et/ou Q, les groupes P et Q ayant la signification indiquée ci-dessus et s et t, indépendamment l'un de l'autre, pouvant prendre les valeurs 0, 1 ou 2,
R¹ et R² représentent, indépendamment l'un de l'autre, un groupe alkyle en C₁ à C₁₆, pouvant être substitué par un ou plusieurs radicaux alkyle en C₁ à C₂₀, groupes alcoxy en C₁ à C₄, atomes d'halogène, groupes sulfo, groupes carboxyle ou groupements de formule - (CH₂)_{w}-COOH, - (CH₂)_{w}-SO₃H, - (CH₂)_{w}-PO₃H₂, -(CH₂)_{w}-OH, w représentant un nombre entier de 0 à 5 et les groupes acides indiqués pouvant également se présenter sous la forme de sel et
X⁻ et Y⁻ représentent, indépendamment l'un de l'autre, R³SO₄⁻ et SO₄²⁻, R³ représentant un atome d'hydrogène, un groupe alkyle en C₁ à C₈ ou aryle en C₆ à C₁₈, le cas échéant substitué par un groupe alkyle en C₁ à C₄, excepté le méthosulfate.

2. Composés selon la revendication 1,
**caractérisés en ce que**
R¹ et R² représentent respectivement un groupe alkyle en C₁ à C₁₆.

3. Composés selon la revendication 1,
**caractérisés en ce que**
R¹ et R² représentent respectivement un groupe méthyle.

4. Composés selon la revendication 1,
**caractérisés en ce que**
A¹, A², A³ et A⁴, indépendamment l'un de l'autre, représentent respectivement un radical alkylène en C₂ à C₄.

5. Composés selon la revendication 1,
**caractérisés en ce que**
A¹ et A² représentent un radical alkylène en C₃, et A³ et A⁴ représentent un radical alkylène en C₂.

6. Composés selon la revendication 1,
**caractérisés en ce que**
X⁻ et Y⁻ représentent un ion R³SO₄⁻, HSO₄⁻ et/ou SO₄²⁻, R³ représentant un atome d'hydrogène ou un groupe alkyle en C₁ à C₈.

7. Composés selon la revendication 1, **caractérisés en ce que** X⁻ et Y⁻ représentent un ion HSO₄⁻.

8. Procédé de préparation de composés selon la revendication 1, dans lesquels A¹, A², A³, A⁴, E¹, R², X et Y ont les significations indiquées dans la revendication
**caractérise en ce qu'**
on convertit une polyamine de formule (3) 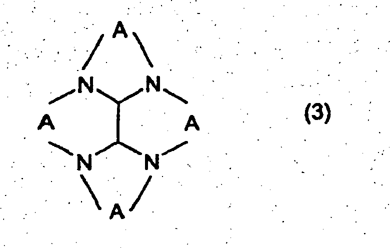 dans laquelle A a les significations indiquées ci-dessus pour A¹, A², A³ et A⁴ avec un composé de formule 4a ou 4b
R¹-Z (4a)
ou
R²-Z (4b)
dans lesquels R¹ et R² ont les significations indiquées ci-dessus et Z a la même signification que X et Y.

9. Procédé selon la revendication 9,
**caractérisé en ce qu'**
on procède à la conversion en présence du composé des formules 4a ou 4b en tant que solvant.

10. Procédé selon la revendication 9,
**caractérisé en ce qu'**
on utilise, pour chaque litre de solvant, de 0,01 à 5,0 moles, de préférence de 0,05 à 3,5 moles et plus préférablement, de 0,1 à 2,0 moles de composé de formule 3.

11. Procédé selon la revendication 9,
**caractérisé en ce qu'**
on procède à la conversion à une température de 0 à 200°C, de préférence de 10 à 150°C.
